# EUROPÄISCHE PATENTSCHRIFT

(11) **EP 0 713 674 B1**
(45) Veröffentlichungstag und Bekanntmachung des Hinweises auf die Patenterteilung: **29.01.2003**
(21) Anmeldenummer: 95117724.5
(22) Anmeldetag: 09.11.1995
(51) Int. Cl.: A61B 5/097, A61B 5/085

(54) **Aufsatz**
Attachment
Accessoire de connection

(30) Priorität: 10.11.1994 DE 4440161
(43) Veröffentlichungstag der Anmeldung: 29.05.1996
(73) Patentinhaber: Müller & Sebastiani Elektronik-GmbH, 81377 München (DE)
(72) Erfinder: Müller, Peter, D-80798 München (DE); Sebastiani, Oscar, D-80469 München (DE)
(74) Vertreter: Manitz, Finsterwald & Partner

(56) Entgegenhaltungen:
- DE-U- 9 113 996
- DE-U- 9 200 635
- FR-A- 2 268 503
- KOPP A ET AL: "RHINOMANOMETRISCHER MESSPLATZ MIT DIGITALANZEIGE" MEDIZINTECHNIK, Bd. 26, Nr. 2, Juni 1986, BERLIN,DE, Seite 55/56 XP000026791

## Beschreibung

Die Erfindung betrifft einen Aufsatz und einen Meßkopf zur Verwendung in der Rhinomanometrie.

Im Rahmen der Allergiediagnostik werden Allergietests durchgeführt, in denen die allergischen Reaktionen eines Patienten auf bestimmte Stoffe untersucht werden. In Hauttests werden diese Stoffe auf die Haut des Patienten aufgebracht, und aus den Reaktionen der Haut wird auf das Vorliegen einer Allergie auf den jeweiligen Stoff geschlossen. Obwohl auf diese Weise gleichzeitig viele verschiedene Allergene getestet werden können, erlaubt dieses Verfahren keine quantitativen Aussagen über die Stärke einer vorhandenen Allergie.

Bei der nasalen Allergenprovokation wird das zu testende Allergen durch ein Nasenloch auf die Nasenschleimhaut aufgebracht und anschließend über die Messung des Nasenflusses der Nasenwiderstand gemessen (Rhinomanometrie). Eine allergische Reaktion der provozierten Nasenschleimhaut führt zu einer Verengung des betreffenden Nasenlochs und ist somit durch eine Erhöhung des Nasenwiderstands nachzuweisen. Zusätzlich erlaubt das Ausmaß der Widerstandserhöhung eine Aussage über die Stärke der Allergie. Eine sich unmittelbar anschließende Untersuchung eines weiteren Allergens an demselben Nasenloch ist allerdings nicht möglich, da eine längere Beruhigungszeit der Nasenschleimhaut eingehalten werden muß.

Aus dem deutschen Gebrauchsmuster DE-U-9 200 635.3 ist auch ein Aufsatz und Meßkopf, wie im Oberbegriff des Anspruchs 1 bescrieben, bekannt.

Aus dem deutschen Gebrauchsmuster G 91 13 996 U1 sind Zubehörteile für Lungenfunktionsgeräte (Spirometer) zum Messen des Nasenflusses bei der nasalen Allergenprovokation bekannt. Dieses Zubehör betrifft eine Atemmaske und einen Silikonschlauch. Bei Benutzung der Atemmaske wird zunächst das eine Nasenloch des Patienten verstopft und das andere Nasenloch jeweils mit dem zu untersuchenden Allergen provoziert.

Anschließend wird dem Patienten die Atemmaske aufgesetzt, diese mit dem Lungenfunktionsgerät verbunden und die Messung durchgeführt. Als Alternative zu der Atemmaske dient der Silikonschlauch zur direkten Koppelung des provozierten Nasenlochs mit dem Lungenfunktionsgerät.

Nachteilig bei diesen bekannten Zubehörteilen ist, daß zur Untersuchung eines weiteren Allergens mit Hilfe des anderen Nasenlochs des Patienten im Fall der Verwendung der Atemmaske das bisher nicht geprüfte und verstopfte Nasenloch freigemacht und anschließend das zuvor provozierte Nasenloch seinerseits verstopft werden muß, was ein zeitaufwendiges Abnehmen und erneutes Aufsetzen der Atemmaske erfordert. Im Fall der Verwendung des Silikonschlauchs muß dieser vor der Untersuchung des zweiten Allergens erst mit dem noch ungeprüften Nasenloch verbunden und das bereits geprüfte Nasenloch verstopft werden, was wie im Fall der Verwendung der Atemmaske zu einer störenden Verzögerung des Allergietests führt.

Es ist eine Aufgabe der Erfindung, eine Vorrichtung zur Verwendung in der Rhinomanometrie zu schaffen, mit der auf einfache Weise allergische Reaktionen eines Patienten auf verschiedene Allergene quantitativ und in kurzer Zeit bestimmt werden können.

Diese Aufgabe wird erfindungsgemäß durch die Merkmale des Anspruchs 1 gelöst.

Durch die Erfindung wird ein Meßkopfaufsatz geschaffen, der sich aufgrund der in seinem Inneren getrennt verlaufenden und wahlweise versperrbaren Kanäle dadurch auszeichnet, daß während des Allergietests schnell und problemlos eine Umstellung von einem Nasenloch auf das andere vorgenommen werden kann, insbesondere ohne daß ein Nasenloch verstopft bzw. eine Schlauchverbindung gelöst und neu hergestellt werden muß.

Das Vorsehen eines Drehschiebers zur selektiven Versperrung der Kanäle ermöglicht ein besonders einfaches und schnelles Umstellen des erfindungsgemäßen Aufsatzes, so daß zeitaufwendige Maßnahmen zur Vorbereitung der Untersuchung eines zweiten Allergens entfallen und somit zwei Allergene innerhalb kürzester Zeit getestet werden können.

Gemäß einer bevorzugten Ausführungsform der Erfindung ist vorgesehen, dass der Aufsatz aus einem im Wesentlichen zylindrischen Verbindungsteil mit einem zur Verschraubung mit dem Messkopf dienenden Gewindeabschnitt, einem im wesentlichen zylindrischen Mittelteil sowie einem sich patientenseitig an den Mittelteil anschließenden und insbesondere einen Abschnitt eines Torus darstellenden Anschlussteil aufgebaut ist, wobei der Verbindungsteil, der Mittelteil und der Anschlussteil einen im Wesentlichen kreisförmigen Querschnitt gleichen Durchmessers aufweisen, der größer als der Durchmesser des im wesentlichen kreisförmigen Querschnitts des Gewindeabschnitts ist.

Ferner ist es bevorzugt, wenn der Durchmesser des im Wesentlichen kreisförmigen Querschnitts des Verbindungsteils, des Mittelteils und des Anschlussteils etwa 6cm und derjenige des Gewindeabschnitts etwa 5,5cm beträgt.

Gemäß einer weiteren Variante der Erfindung ist vorgesehen, dass der Mittelteil und der Anschlussteil einstückig miteinander ausgebildet sind und der Verbindungsteil mit dem Mittelteil verbindbar und insbesondere verschraubbar ist.

Ferner wird vorgeschlagen, dass der Gewindeabschnitt mit einer Umfangsnut zur Aufnahme einer Dichtung versehen ist.

Gemäß einem weiteren bevorzugten Ausführungsbeispiel treten die Kanäle patientenseitig am Anschlussteil aus dem Aufsatz aus.

Des Weiteren kann erfindungsgemäß vorgesehen sein, dass der Mittelteil des Aufsatzes Mittel zur Versperrung der Kanäle aufweist.

Eine weitere Ausführungsform der Erfindung schlägt vor, dass der Drehschieber und die zu seiner Aufnahme dienende Ausnehmung im Mittelteil des Aufsatzes angeordnet sind.

Des Weiteren ist es bevorzugt, wenn der Aufsatz im Wesentlichen zylinderförmig ist und einen Radius von etwa 3cm und eine Länge von etwa 6cm aufweist.

Ferner kann vorgesehen sein, dass der Aufsatz aus Kunststoff, insbesondere im Spritzgussverfahren aus ABS hergestellt ist.

Vorzugsweise ist der Aufsatz einstückig mit dem Messkopf ausgebildet.

Gemäß einer weiteren bevorzugten Ausführungsform der Erfindung ist vorgesehen, dass der Aufsatz mit einem Nasenansatz koppelbar ist, der einen Hals, einen auf dem Hals angeordneten und vorzugsweise einstückig mit diesem ausgebildeten Auflageabschnitt und einen insbesondere abnehmbar auf dem Auflageabschnitt vorgesehenen Kontaktabschnitt umfasst, wobei jeweils im Hals bzw. im Kontaktabschnitt Anschlüsse bzw. zur Koppelung des Nasenansatzes an den Aufsatz bzw. mit den Nasenlöchern eines Patienten vorgesehen sind, die durch im wesentlichen in vertikaler Richtung durch den Nasenansatz verlaufende Kanäle verbunden sind, und wobei die dem Patienten zugewandte Seite des Kontaktabschnitts und vorzugsweise auch des Auflageabschnitts des Nasenansatzes zur Anpassung an den Oberlippenbereich des Patienten eine Ausnehmung mit im wesentlichen halbkreisförmigem Querschnitt aufweist.

Ferner kann vorgesehen sein, dass die Austrittsöffnungen der Kanäle am Kontaktabschnitt des Nasenansatzes jeweils eine Fläche aufweisen, die ungefähr gleich derjenigen eines Nasenlochs des jeweiligen Patienten ist.

Des Weiteren ist es bevorzugt, wenn die horizontale Querschnittsfläche der Kanäle des Nasenansatzes jeweils in Richtung des unteren Endes des Halses abnimmt.

Weitere Ausführungsformen der Erfindung sind in den Unteransprüchen angegeben.

Die Erfindung wird im folgenden beispielhaft anhand der Zeichnung beschrieben; in dieser zeigt:
- Figur 1: eine Seitenansicht eines erfindungsgemäßen Aufsatzes, in welcher der Drehschieber nicht gezeigt ist,
- Figur 2: einen Schnitt A-A durch den erfindungsgemäßen Aufsatz,
- Figur 3: einen Schnitt B-B durch den erfindungsgemäßen Aufsatz,
- Figur 4: einen Drehschieber für den erfindungsgemäßen Aufsatz,
- Figur 5: eine Draufsicht auf den Drehschieber in Richtung des Pfeils C gemäß Figur 4,
- Figur 6: die Wirkungsweise des Drehschiebers in einer ersten Stellung,
- Figur 7: die Wirkungsweise des Drehschiebers in einer zweiten Stellung, und
- Figur 8: eine Seitenansicht einer Ausführungsform einer Nasenmaske, die zur Koppelung mit einem erfindungsgemäßen Aufsatz geeignet ist,
- Figur 9: eine Ansicht der dem Patienten zugewandten Seite der Nasenmaske,
- Figur 10: eine Draufsicht auf die Nasenmaske,
- Figur 11: eine Seitenansicht einer Ausführungsform eines Nasenansatzes, der zur Koppelung mit einem erfindungsgemäßen Aufsatz geeignet ist,
- Figur 12: eine Draufsicht auf den Nasenansatz, und
- Figur 13: eine Ansicht der dem Patienten zugewandten Seite des Nasenansatzes.

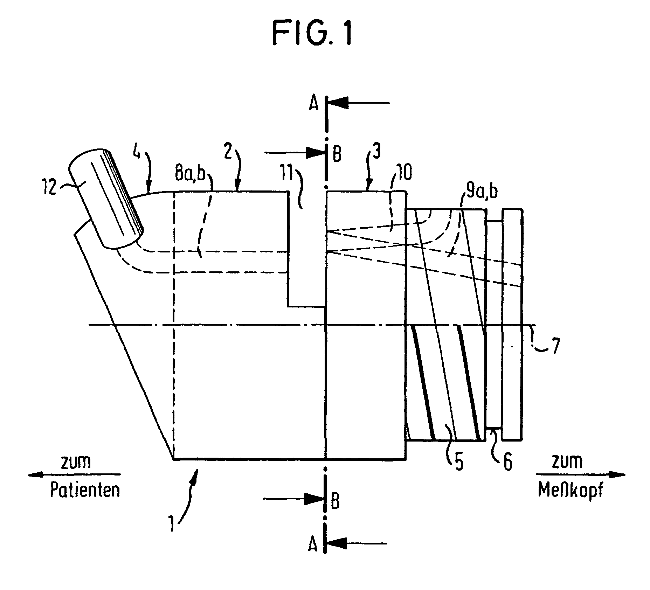

Nach Figur 1 besteht der erfindungsgemäße Aufsatz 1 aus einem zylindrischen Mittelteil 2, einem zylindrischen Verbindungsteil 3 mit einem ebenfalls zylindrischen Gewindeabschnitt 5 kleineren Querschnitts sowie einem einen Abschnitt eines Torus darstellenden Anschlußteil 4. Der Gewindeabschnitt 5 dient zur Verschraubung des Aufsatzes 1 mit einem nicht dargestellten Meßkopf und ist mit einer Umfangsnut 6 versehen, die zur Aufnahme einer Dichtung dient.

Die kreisförmigen Querschnittsflächen des Mittelteils 2, des Anschlußteils 4 und des Verbindungsteils 3, deren Mittelpunkte auf einer die Aufsatzachse 7 bildenden Geraden liegen, sind gleich groß. Da zum einen der Querschnitt des Gewindeabschnitts 5 mit einem Durchmesser von vorzugsweise etwa 5,5cm nicht wesentlich kleiner als derjenige der anderen Teile des Aufsatzes 1 ist, und zum anderen der Anschlußteil 4 nur einen relativ kurzen Torusabschnitt darstellt, ist der Aufsatz 1 ein im wesentlichen zylinderförmiger und kompakter Gegenstand, der bevorzugt einen Radius von etwa 3cm und eine Länge von etwa 6cm aufweist und vorzugsweise aus Kunststoff, insbesondere aus ABS, beispielsweise im Spritzgußverfahren hergestellt ist.

Vorzugsweise sind der Anschlußteil 4 und der Mittelteil 2 einstückig miteinander ausgebildet, wohingegen der Verbindungsteil 3 durch nicht dargestellte Schrauben mit dem Mittelteil 2 verschraubbar ist. Ebenso können jedoch auch alle Teile 2, 3, 4 des Aufsatzes einstückig miteinander ausgebildet werden.

In dem Aufsatz 1 sind mehrere Kanäle ausgebildet, die aus zwei identischen patientenseitigen Kanalabschnitten 8a, b, zwei ebenfalls identischen meßkopfseitigen Kanalabschnitten 9a, b und einem meßkopfseitigen zusätzlichen Kanal 10 bestehen. Im Mittelteil 2 des Aufsatzes 1 ist an dessen meßkopfseitigen Ende eine senkrecht zur Aufsatzachse 7 verlaufende Ausnehmung 11 ausgebildet. Die Kanalabschnitte 9a, b verlaufen von der Ausnehmung 11 durch den Verbindungsteil 3 zu dessen meßkopfseitigen Ende, wo sie in den Meßkopf münden, wenn dieser mit dem Aufsatz 1 verschraubt ist. Der zusätzliche Kanal 10 verläuft ebenfalls im Verbindungsteil 3 von der Ausnehmung 11 zum Gewindeabschnitt 5, wo er aus dem Aufsatz 1 austritt. In diesem Bereich kann ein mit dem Meßkopf verbundener, nicht gezeigter Drucksensor angeordnet werden. Wird dieser Drucksensor nicht verwendet, kann der Kanal 10 dort mit einem nicht dargestellten Stopfen verschlossen werden.

Die Kanalabschnitte 8a, b verlaufen von der Ausnehmung 11, in die sie bezüglich der Kanalabschnitte 9a, b versetzt münden, durch den Mittelteil 2 zum Anschlußteil 4, wo sie aus dem Aufsatz 1 austreten und mit Anschlußhülsen 12 versehen sind, über die sie beispielsweise direkt oder mittels Schlauchverbindungen oder über einen geeigneten Ansatzstutzen strömungstechnisch mit den Nasenlöchern eines Patienten koppelbar sind. Die Anschlußhülsen 12 verlaufen in einer Ebene liegend im wesentlichen parallel zur patientenseitigen Stirnfläche des Anschlußteils 4 und schließen mit den im Mittelteil 2 verlaufenden Kanalabschnitten 8a, b bevorzugt einen Winkel von etwas mehr als 90° ein.

Über die patientenseitigen Kanalabschnitte 8a, b und die meßkopfseitigen Kanalabschnitte 9a, b sind die Nasenlöcher des Patienten mit dem Meßkopf strömungstechnisch koppelbar. Die Ausnehmung 11 dient dabei zur Aufnahme eines in Figur 1 nicht dargestellten und nachstehend beschriebenen Drehschiebers, mit dem eine selektive Versperrung der einzelnen Kanäle möglich ist.

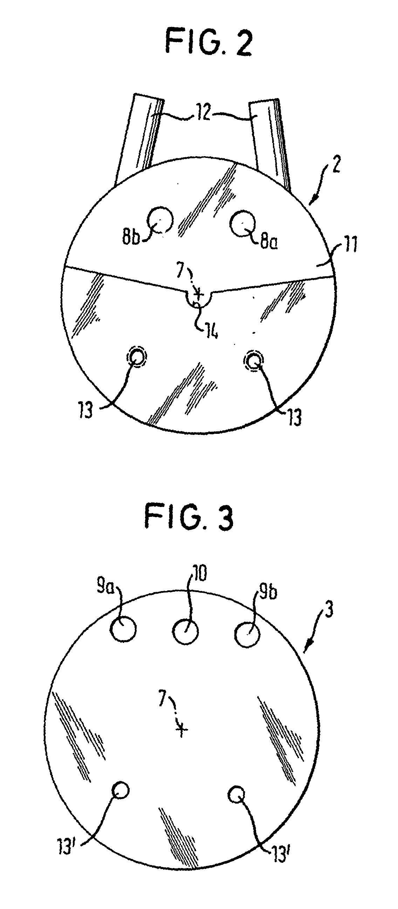

Figur 2 zeigt in einem Schnitt durch den Aufsatz senkrecht zu dessen Achse eine Ansicht des meßkopfseitigen Endes des Mittelteils 2. Zu erkennen sind die unter einem kleinen Winkel aufeinander zu verlaufenden Anschlußhülsen 12 sowie mit einem Innengewinde versehene Bohrungen 13, die zur Verschraubung des Mittelteils 2 mit dem Verbindungsteil 3 dienen.

Die im Mittelteil 2 ausgebildete Ausnehmung 11 weist einen kreissektorförmigen Querschnitt auf, der einen Winkelbereich von vorzugsweise etwa 160° abdeckt. Zusätzlich ist die Ausnehmung 11 mit einem im wesentlichen halbkreisförmigen Führungsausschnitt 14 versehen, dessen Mittelpunkt mit demjenigen des die Ausnehmung 11 bildenden Kreissektors zusammenfällt.

Die Ausnehmung 11, die in sie mündenden patientenseitigen Kanalabschnitte 8a, b, die Anschlußhülsen 12 sowie die Bohrungen 13 sind jeweils beidseitig einer den Aufsatz entlang seiner Achse 7 schneidenden vertikalen Ebene in gleicher Entfernung zu dieser angeordnet.

Figur 3 zeigt in der gleichen Schnittebene wie Figur 2 eine Ansicht des patientenseitigen Endes des Verbindungsteils 3. Zu erkennen sind entsprechend den Bohrungen 13 angeordnete, zur Verschraubung des Verbindungsteils 3 mit dem Mittelteil 2 dienende Schrauben aufnehmende Bohrungen 13' sowie die meßkopfseitigen Kanalabschnitte 9a, b und der zusätzliche Kanal 10, die an der in Figur 3 gezeigten Schnittebene in die Ausnehmung 11 des Mittelteils münden. Im Vergleich zu den Kanalabschnitten 8a, b sind dabei die Mündungen der Kanalabschnitte 9a, b in einer größeren Entfernung zur Aufsatzachse 7 angeordnet. Entsprechend Figur 2 sind die meßkopfseitigen Kanalabschnitte 9a, b sowie die Bohrungen 13' jeweils beidseitig einer den Aufsatz entlang seiner Achse 7 schneidenden vertikalen Ebene in gleicher Entfernung zu dieser angeordnet, wobei der zusätzliche Kanal 10 so angeordnet ist, daß seine Achse in dieser Ebene liegt.

Wenn das Verbindungsteil 3 mit dem Mittelteil 2 verschraubt ist, sind somit die Kanäle 8a, b, 9a, b und 10 sowie die Ausnehmung 11 und die Anschlußhülsen 12 symmetrisch bezüglich einer den Aufsatz entlang seiner Achse 7 schneidenden vertikalen Ebene angeordnet, so daß der Aufsatz bezüglich dieser Ebene vollkommen symmetrisch aufgebaut ist.

Es ist auch möglich, jeweils anstelle eines Kanalabschnitts eine Mehrzahl kleinerer Kanäle vorzusehen, die dann jeweils gemeinsam an dem entsprechenden Bereich in die Ausnehmung münden.

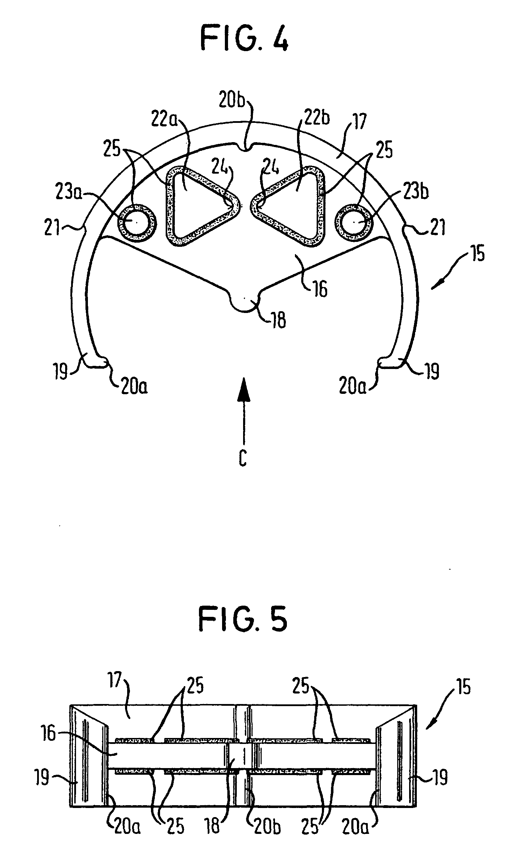

Figur 4 zeigt einen zur selektiven Versperrung der im Aufsatz angeordneten Kanäle dienenden Drehschieber 15. Dieser besteht aus einem im wesentlichen kreissektorförmigen Funktionsbereich 16 und einem Betätigungsbereich 17, die vorzugsweise einstückig miteinander ausgebildet sind. Der Betätigungsbereich 17 ist als ein kreisbogenförmig gekrümmtes, an der kreisbogenförmigen Seite des Funktionsbereiches 16 angebrachtes Bandelement ausgebildet, das sich senkrecht zum Funktionsbereich 16 erstreckend beidseitig über diesen vorsteht. Die Gesamtausdehnung des Betätigungsbereiches 17 senkrecht zum Funktionsbereich 16 ist dabei größer als die Ausdehnung der Ausnehmung im Mittelteil des Aufsatzes in Richtung der Aufsatzachse.

Der Radius des kreissektorförmigen Funktionsbereiches 16 entspricht demjenigen der Ausnehmung im Mittelteil des Aufsatzes, wobei aber der Funktionsbereich 16 des Drehschiebers 15 mit vorzugsweise etwa 130° einen kleineren Winkelbereich abdeckt als die Ausnehmung. Der Funktionsbereich 16 weist einen im wesentlichen halbkreisförmigen Ansatz 18 auf, der dem halbkreisförmigen Führungsausschnitt der Ausnehmung entspricht.

Der Betätigungsbereich 17 des Drehschiebers 15 ist entsprechend dem Radius der kreissektorförmigen Ausnehmung im Mittelteil des Aufsatzes gekrümmt und erstreckt sich über einen Winkelbereich von mehr als 180°. Die freien Enden 19 des Betätigungsbereiches 17 sind vorzugsweise flexibel ausgebildet und mit radial nach innen vorstehenden Ansätzen 20a versehen. Außerdem ist der Betätigungsbereich 17 radial innen an seinem Scheitelpunkt auf beiden Seiten des Funktionsbereiches 16 mit senkrecht zu diesem verlaufenden Ansätzen 20b versehen.

Die sich in Umfangsrichtung über den Funktionsbereich 16 des Drehschiebers 15 hinaus erstreckenden Abschnitte des Betätigungsbereiches 17 sind von geringerer radialer Ausdehnung als der direkt mit dem Funktionsbereich 16 verbundene Abschnitt des Betätigungsbereiches 17, wobei der Übergangsbereich jeweils durch Stufen 21 gebildet ist, die die Griffigkeit der äußeren Oberfläche des Betätigungsbereiches 17 verbessern.

In dem Funktionsbereich 16 des Drehschiebers 15 sind zwei Öffnungen 22a, b und zwei Sperrelemente 23a, b angeordnet, wobei letztere vorzugsweise durch den Funktionsbereich 16 selbst gebildet sind. Der Querschnitt der kreisförmigen Sperrelemente 23a, b entspricht dabei mindestens dem Querschnitt der im Aufsatz verlaufenden Kanäle. Die Öffnungen 22a, b weisen jeweils einen Querschnitt eines im wesentlichen gleichseitigen Dreiecks mit abgerundeten Ecken 24 auf, deren Krümmungsradius jeweils demjenigen der im Aufsatz verlaufenden Kanäle entspricht. Sowohl die Sperrelemente 23a, b als auch die Öffnungen 22a, b sind mit umlaufenden Dichtungen 25 versehen, die vorzugsweise in jeweils im Randbereich der Öffnungen 22a, b und Sperrelemente 23a, b ausgebildeten Nuten angeordnet sind, deren Tiefe geringer ist als der Querschnitt der Dichtungen 25.

Figur 5 zeigt eine Draufsicht auf den Drehschieber 15 in Richtung des in Figur 4 gezeigten Pfeils C. Am Betätigungsbereich 17 sind die an seinen freien Enden 19 ausgebildeten Ansätze 20a sowie die Ansätze 20b, und am Funktionsbereich 16 der im wesentlichen halbkreisförmige Ansatz 18 sowie die um die Öffnungen und Sperrelemente umlaufenden Dichtungen 25 zu erkennen. Die Ausdehnung des Betätigungsbereiches 17 senkrecht zum Funktionsbereich 16 nimmt in seinen sich in Umfangsrichtung über den Funktionsbereich 16 des Drehschiebers 15 hinaus erstreckenden Abschnitten in Richtung der freien Enden 19 stetig ab.

Im folgenden wird die Funktionsweise des erfindungsgemäßen Aufsatzes beschrieben.

Zur Messung des Nasenwiderstands wird der Aufsatz 1 zunächst über den Gewindeabschnitt 5 am Verbindungsteil 3 mit dem Meßkopf verschraubt. Nach der Provokation der Nasenlöcher des Patienten mit den zu untersuchenden Allergenen werden die Nasenlöcher beispielsweise über einen speziellen Ansatzstutzen mit den Anschlußhülsen 12 am Anschlußteil 4 des Aufsatzes 1 strömungstechnisch gekoppelt. Über die im Aufsatz 1 ausgebildeten Kanäle und den in die Ausnehmung 11 eingesetzten Drehschieber 15 sind die Nasenlöcher somit auf eine nachstehend beschriebene Weise selektiv mit dem Meßkopf koppelbar.

Beim Einsetzen des Drehschiebers 15 in die Ausnehmung 11 werden die vorzugsweise flexibel ausgebildeten freien Enden 19 des kreisbogenförmig gekrümmten Betätigungsbereiches 17 radial nach außen gedrückt und gelangen in ihre Ausgangslage zurück, wenn der Drehschieber 15 mit seinem am Funktionsbereich 16 ausgebildeten Ansatz 18 im Führungsausschnitt 14 gelagert ist. In dieser Position liegt der Mittelpunkt des im wesentlichen halbkreisförmigen Ansatzes 18 auf der Aufsatzachse 7. Da der Radius des Funktionsbereiches 16 demjenigen der Ausnehmung 11 und damit des Mittelteils 2 entspricht, liegt der Betätigungsbereich 17 mit seinen Ansätzen 20a und 20b auf der Oberfläche des zylindrischen Mittelteils 2 auf und erstreckt sich manschettenartig über einen Winkelbereich von mehr als 180° über einen Umfangsabschnitt des Aufsatzes 1. Dadurch wird der Drehschieber 15 auf vorteilhafte Weise klammerartig am Aufsatz 1 gehalten.

Da der Funktionsbereich 16 des Drehschiebers 15 mit vorzugsweise 130° einen kleineren Winkelbereich abdeckt als mit vorzugsweise etwa 160° die Ausnehmung 11, steht für die Drehung des Drehschiebers 15 im Aufsatz 1 mit seinem im Führungsausschnitt 14 gelagerten Ansatz 18 ein Winkelbereich von etwa 30° zur Verfügung. Beim Drehen des Drehschiebers 15 gleitet dieser lediglich mit seinen Ansätzen 20a und 20b auf dem Umfang des Aufsatzes 1, und die Reibung ist somit auf ein Minimum begrenzt.

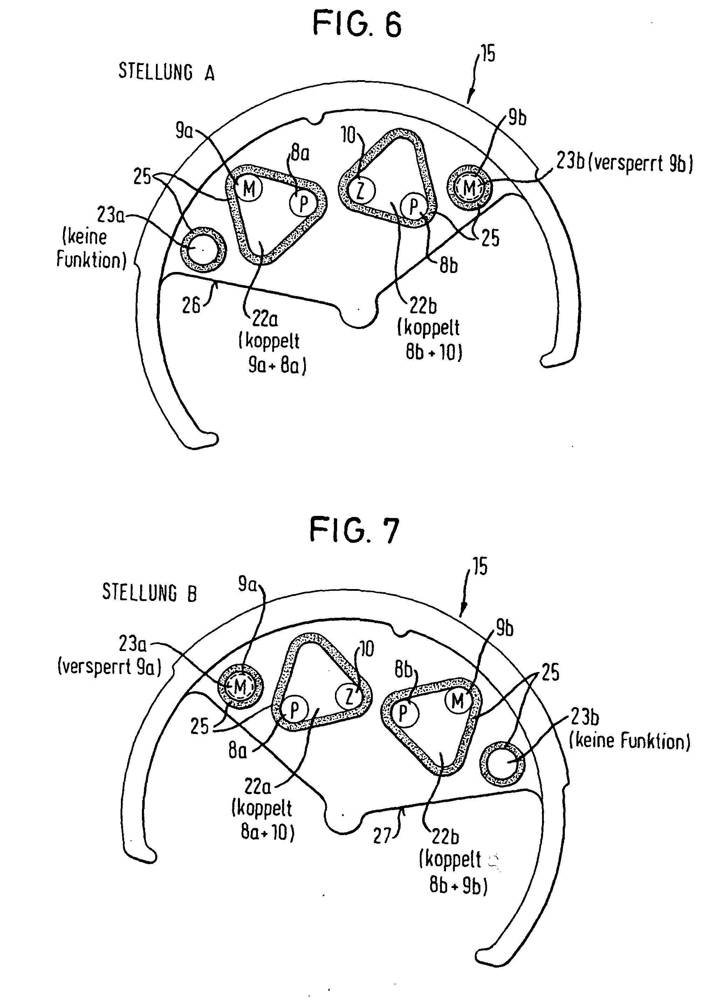

Die Wirkungsweise des Drehschiebers 15 im Aufsatz 1 wird im folgenden anhand der Figuren 6 und 7 beschrieben.

In den Figuren 6 und 7 bezeichnen die mit einem P versehenen Kreise die patientenseitigen Kanalabschnitte 8a, b, die mit einem M versehenen Kreise die meßkopfseitigen Kanalabschnitte 9a, b, und die mit einem Z versehenen Kreise den zusätzlichen Kanal 10, wobei ein gestrichelt gezeichneter Kreis bedeutet, daß der jeweilige Kanalabschnitt von einem Sperrelement 23a, b versperrt ist, und ein durchgezogener Kreis einen von einer Öffnung 22a, b freigegebenen Kanalabschnitt andeutet.

In der in Figur 6 gezeigten Stellung A des Drehschiebers 15 ist dieser um die Aufsatzachse gegen den Uhrzeigersinn soweit gedreht, daß die in Drehrichtung vorne liegende Kante 26 des Funktionsbereiches 16 an das entsprechende Ende der im Mittelteil vorgesehenen Ausnehmung stößt. In dieser Stellung münden der meßkopfseitige Kanalabschnitt 9a und der patientenseitige Kanalabschnitt 8a in die Öffnung 22a, wodurch das entsprechende Nasenloch des Patienten mit dem Meßkopf strömungstechnisch gekoppelt ist. Eine Messung des Nasenwiderstands bezüglich dieses Nasenlochs und damit eine quantitative Untersuchung der allergischen Reaktion des Patienten auf das die betreffende Nasenschleimhaut provozierende Allergen wird auf diese Weise ermöglicht.

Gleichzeitig münden in der Stellung A des Drehschiebers 15 der patientenseitige Kanalabschnitt 8b und der zusätzliche Kanal 10 in die andere Öffnung 22b des Drehschiebers 15, wodurch das andere Nasenloch des Patienten strömungstechnisch mit dem zusätzlichen Kanal 10 gekoppelt ist. Mittels eines im Bereich des Austritts des zusätzlichen Kanals 10 aus dem Aufsatz 1 angeordneten und den Kanal 10 abschließenden Drucksensors lassen sich so beispielsweise Vergleichsmessungen mit diesem Nasenloch durchführen. Der Ausgang des zusätzlichen Kanals 10 kann aber auch mit einem Stopfen verschlossen werden, was einer Verstopfung des gerade nicht untersuchten Nasenlochs gleichkommt. In jedem Fall sind jedoch die beiden Nasenlöcher des Patienten stets vollständig strömungstechnisch voneinander getrennt.

Außerdem versperrt in Stellung A das Sperrelement 23b des Drehschiebers 15 den meßkopfseitigen Kanalabschnitt 9b, so daß in den Meßkopf ausschließlich die vom Patienten durch das zu untersuchende, über die Kanalabschnitte 8a und 9a sowie die Öffnung 22a gekoppelte Nasenloch ausgeatmete Luft strömt und somit zur Untersuchung des betreffenden Allergens eine einwandfreie Messung des Widerstandes dieses Nasenlochs gewährleistet ist. Das Sperrelement 23a ist in der in Figur 6 gezeigten Drehschieberstellung ohne Funktion.

Ist die in Stellung A des Drehschiebers erfolgende Messung beendet, wird der Drehschieber 15 um die Aufsatzachse im Uhrzeigersinn gedreht, bis die jetzt vorne liegende andere Kante 27 des Funktionsbereiches 16 an das entsprechende Ende der Ausnehmung 11 stößt und die in Figur 7 gezeigte Stellung B des Drehschiebers 15 erreicht ist. Aufgrund des symmetrischen Aufbaus sowohl des Aufsatzes 1 als auch des Drehschiebers 15 bezüglich einer den Aufsatz 1 entlang seiner mit der Drehachse des Drehschiebers 15 zusammenfallenden Achse schneidenden vertikalen Ebene bewirkt der Drehschieber 15 in dieser Stellung eine selektive Versperrung und Freigabe der einzelnen Kanalabschnitte prinzipiell wie in Stellung A, wobei jedoch in Stellung B nun das zuerst untersuchte Nasenloch mit dem zusätzlichen Kanal 10 und das andere, zuvor nicht untersuchte Nasenloch des Patienten mit dem Meßkopf strömungstechnisch gekoppelt ist.

Entsprechend der Stellung A koppelt in Stellung B also die Öffnung 22a den patientenseitigen Kanalabschnitt 8a mit dem zusätzlichen Kanal 10, während das Sperrelement 23a den meßkopfseitigen Kanalabschnitt 9a versperrt. Die Öffnung 22b koppelt nun den patientenseitigen Kanalabschnitt 8b mit dem meßkopfseitigen Kanalabschnitt 9b und somit das zuvor nicht untersuchte Nasenloch mit dem Meßkopf, so daß nun eine Messung des Widerstands dieses Nasenlochs zur Untersuchung der Reaktion auf das zweite Allergen möglich ist. In Stellung B hat das Sperrelement 23b des Drehschiebers 15 keine Funktion.

Im Fall der Ausbildung einer Mehrzahl kleinerer Kanäle jeweils anstelle eines Kanalabschnitts sind dann in beiden Stellungen A und B des Drehschiebers 15 die kleinen Kanäle jeweils gemeinsam durch das jeweilige Sperrelement 23a, b versperrbar.

Die um die Öffnungen 22a, b und Sperrelemente 23a, b des Drehschiebers 15 umlaufenden Dichtungen 25 garantieren in beiden Drehschieberstellungen eine vollständige strömungstechnische Trennung der jeweils nicht zu koppelnden Kanalabschnitte.

Sowohl in der Stellung A als auch der Stellung B ermöglicht die erfindungsgemäße Ausbildung der Öffnungen 22a, b als gleichseitige Dreiecke mit abgerundeten Ecken, deren Krümmungsradius demjenigen der zu koppelnden Kanalabschnitte entspricht, zusammen mit der versetzten Anordnung der Mündungen der Kanalabschnitte in der Ausnehmung eine Minimierung des zur Verstellung des Drehschiebers 15 zwischen den Stellungen A und B erforderlichen Drehwinkels.

Durch die Erfindung wird somit auf vorteilhafte Weise die Möglichkeit geschaffen, auf dem Gebiet der nasalen Allergenprovokation eine Untersuchung der allergischen Reaktionen eines Patienten auf zwei unterschiedliche Allergene quantitativ und in kürzester Zeit ohne einen apparativ bedingten, die Untersuchung unnötig verlängernden Zeitaufwand durchzuführen.

Zusätzlich sind in einer Mittelstellung des Drehschiebers 15 zwischen den Stellungen A und B gleichzeitig die Kanalabschnitte 8a, 9a sowie 8b, 9b jeweils miteinander über die Öffnungen 22a sowie 22b koppelbar, um beide Nasenlöcher des Patienten mit dem Meßkopf strömungstechnisch zu koppeln. Der zusätzliche Kanal 10 ist dabei vom Funktionsbereich 16 des Drehschiebers 15 versperrt, wobei die beiden Sperrelemente 23a, b keine Funktion haben.

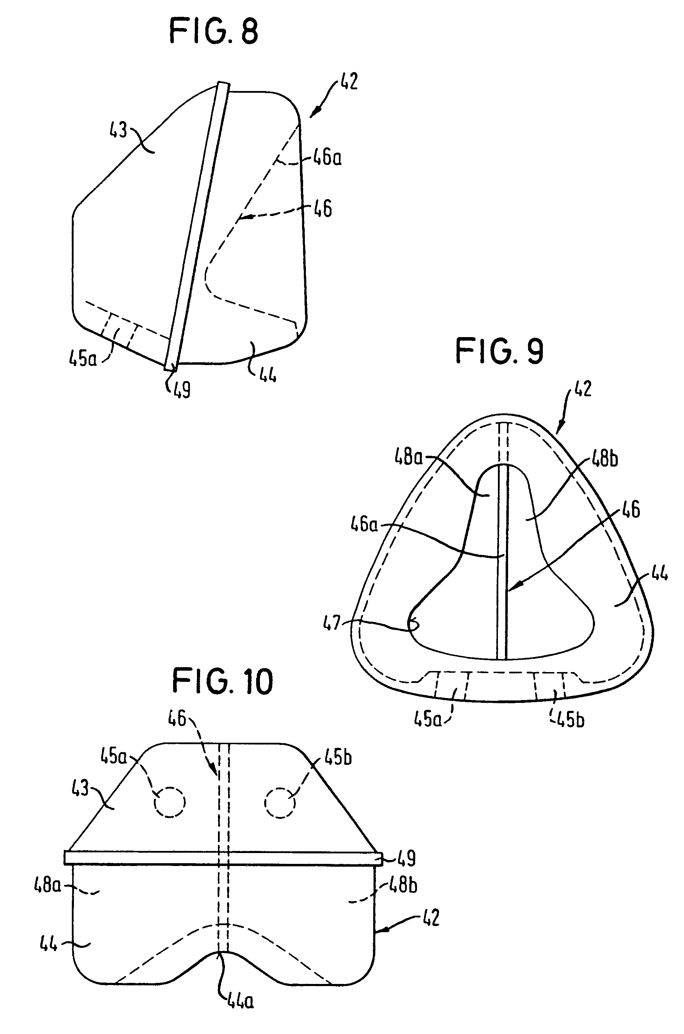

In den Figuren 8 bis 10 ist eine Ausführungsform einer Nasenmaske 42 gezeigt, die zur Koppelung mit einer Volumenstrommeßeinrichtung und insbesondere mit dem vorstehend beschriebenen Aufsatz geeignet ist.

Nach Figur 8 weist die Nasenmaske 42 einen Maskenabschnitt 43 auf, der an seinem unteren Bereich mit Anschlüssen 45a, b versehen und an seiner dem Patienten zugewandten Seite offen ist. Die Anschlüsse 45a, b dienen zur Koppelung der Nasenmaske 42 an den erfindungsgemäßen Aufsatz. An den Randbereich der offenen Seite des Maskenabschitts 43 schließt sich ein Dichtungsabschnitt 44 an, wobei die Verbindung 49 zwischen Maskenabschnitt 43 und Dichtungsabschnitt 44 vorzugsweise so ausgebildet ist, daß der Dichtungsabschnitt 44 auswechselbar am Maskenabschnitt 43 gehalten ist.

Im Inneren der Nasenmaske 42 ist eine sich im wesentlichen vertikal erstreckende und vorzugsweise einstückig mit dem Maskenabschnitt 43 ausgebildete Trennwand 46 angeordnet, von der in Figur 8 lediglich durch eine gestrichelte Linie das patientenseitige Ende 46a dargestellt ist.

Figur 9 zeigt eine Ansicht der dem Patienten zugewandten Seite der Nasenmaske 42. Der Dichtungsabschnitt 44 weist eine Öffnung 47 auf, durch die beim Aufsetzen der Nasenmaske 42 die Nase des Patienten eingeführt wird. Der Umriß der Öffnung 47 ist vorzugsweise an die Form einer Nase angepaßt.

In Figur 9 ist außerdem das patientenseitige Ende 46a der im Inneren der Nasenmaske 42 angeordneten Trennwand 46 zu erkennen, die das Innere der Nasenmaske 42 in zwei Kammern 48a, b unterteilt. In diese Kammern 48a, b mündet jeweils einer der Anschlüsse 45a, b.

Aus Figur 10, die eine Draufsicht auf die Nasenmaske 42 zeigt, ist zu erkennen, daß die Trennwand 46 im wesentlichen senkrecht zu der Verbindung 49 zwischen Maskenabschnitt 43 und Dichtungsabschnitt 44 verläuft und so angeordnet ist, daß die durch die Trennwand 46 entstehenden Kammern 48a, b im Inneren der Nasenmaske 42 ungefähr die gleiche Größe aufweisen. Die Anschlüsse 45a, b sind symmetrisch bezüglich der Trennwand 46 angeordnet.

Figur 10 zeigt außerdem, daß die dem Patienten zugewandte Seite des Dichtungsabschnitts 44 mit einer Ausnehmung 44a versehen ist, durch welche der Dichtungsabschnitt 44 an das Gesicht des Patienten angepaßt ist und somit das Aufsetzen der Nasenmaske 42 verbessert wird.

Nachstehend wird die Benutzung der Nasenmaske 42 im Rahmen der Rhinomanometrie beschrieben.

Im Fall einer Allergiediagnose wird nach erfolgter Allerqenprovokation der Nasenschleimhäute des Patienten die Nasenmaske 42 auf die Nase aufgesetzt. Der Maskenabschnitt 43 besteht vorzugsweise aus einem festen Material hoher Steifigkeit, so daß die Nasenmaske 42 beim Aufsetzen am festen Maskenabschnitt festgehalten werden kann. Der Dichtungsabschnitt 44 ist vorzugsweise aus einem weichen Material, insbesondere Silikon hergestellt, wodurch sich der Dichtungsabschnitt 44 im aufgesetzten Zustand der Nasenmaske 42 optimal an die Gesichtsform des Patienten anpaßt und auf diese Weise die Nasenlöcher am Gesicht des Patienten gegen die Umgebung abdichtet.

Das dem Patienten zugewandte Ende 46a der Trennwand 46 ist nach Figur 9 entsprechend einer Nase geformt, so daß im aufgesetzten Zustand der Nasenmaske 42 in die Kammern 48a, b jeweils ein Nasenloch des Patienten mündet. Auf diese Weise sind die beiden Nasenlöcher vollständig strömungstechnisch voneinander getrennt und jeweils mit einem der ebenfalls in die Kammern 48a, b mündenden Anschlüsse 45a, b gekoppelt.

Durch Koppelung der Anschlüsse 45a, b an eine Volumenstrommeßeinrichtung und insbesondere den erfindungsgemäßen Aufsatz wird im Rahmen der Rhinomanometrie durch die Nasenmaske 42 eine bequeme Untersuchung der Reaktionen des Patienten auf die durch seine Nasenlöcher eingebrachten Allergene ermöglicht.

Die Auswechselbarkeit des Dichtungsabschnitts 44 am Maskenabschnitt 43 gewährleistet einwandfreie hygienische Bedingungen und ermöglicht ferner die Durchführung von aufeinanderfolgenden Allergieuntersuchungen in schneller Abfolge, da der Maskenabschnitt 43 ständig an die Volumenstrommeßeinrichtung oder den Aufsatz gekoppelt verbleiben kann.

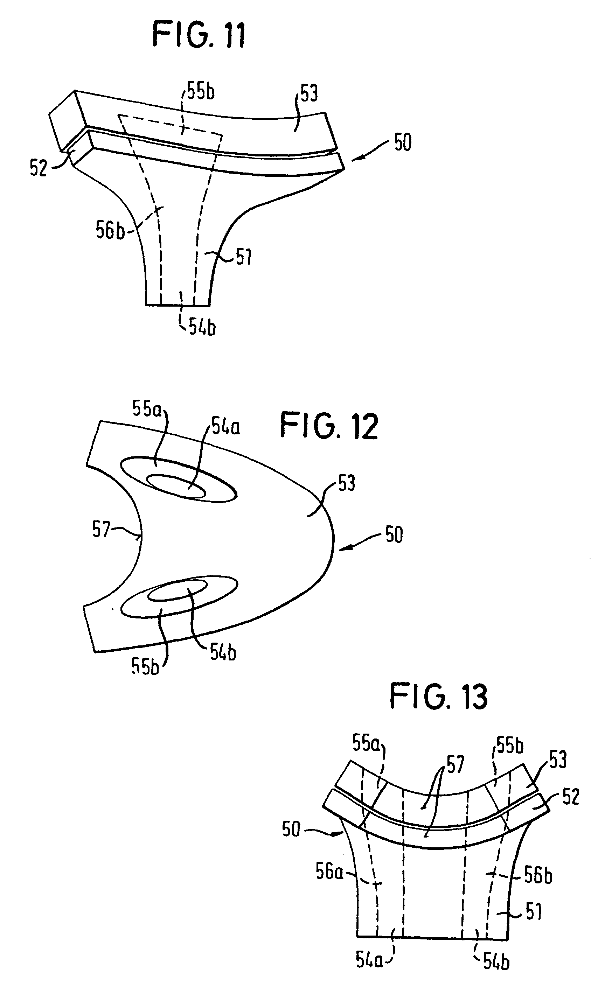

In den Figuren 11 bis 13 ist ein Nasenansatz 50 gezeigt, der eine weitere vorteilhafte Möglichkeit zur Koppelung der Nasenlöcher eines Patienten mit einer Volumenstrommeßeinrichtung und insbesondere dem vorstehend beschriebenen erfindungsgemäßen Aufsatz im Rahmen der Rhinomanometrie bietet.

In der in Figur 11 dargestellten Ausführungsform umfaßt der Nasenansatz 50 einen Hals 51, einen auf dem Hals 51 angeordneten Auflageabschnitt 52 und einen Kontaktabschnitt 53, der abnehmbar auf dem Auflageabschnitt 52 vorgesehen ist. Der Hals 51 und der Auflageabschnitt 52 sind vorzugsweise einstückig miteinander ausgebildet.

Im Nasenansatz 50 sind am unteren Ende des Halses 51 zwei Anschlüsse 54a, b, von denen in Figur 11 lediglich der Anschluß 54b zu erkennen ist, und auf der Oberseite des Kontaktabschnitts 53 zwei weitere Anschlüsse 55a, b vorgesehen, von denen in Figur 11 der Anschluß 55b gezeigt ist. Die am unteren Ende des Halses 51 vorgesehenen Anschlüsse 54a, b dienen zur Koppelung des Nasenansatzes 50 mit der Volumenstrommeßeinrichtung oder dem erfindungsgemäßen Aufsatz und die auf der Oberseite des Kontaktabschnitts 53 vorgesehenen Anschlüsse 55a, b zur Kopplung des Nasenansatzes 50 mit den Nasenlöchern des Patienten. Die Anschlüsse 54a, b und 55a, b sind durch im wesentlichen in vertikaler Richtung durch den Nasenansatz 50 verlaufende Kanäle 56a, b verbunden, von denen in Figur 11 lediglich der Kanal 56b zu erkennen ist.

Der von dem Hals 51 und dem Auflageabschnitt 52 gebildete Bereich des Nasenansatzes 50 ist so geformt, daß seine horizontale Querschnittsfläche vom unteren Ende des Halses 51 zum oberen Ende des Auflageabschnitts 52 so zunimmt, daß zwischen dem Hals 51 des Nasenansatzes 50 und dem Gesicht des Patienten ein Zwischenraum vorhanden ist, wenn die dem Patienten zugewandte Seite des Kontaktabschnitts 53 unterhalb der Nase am Oberlippenbereich des Patienten angesetzt ist.

Figur 12 zeigt eine Draufsicht auf den Nasenansatz 50, in der die beiden auf der Oberseite des Kontaktabschnitts 53 ausgebildeten Anschlüsse 55a, b und die am unteren Ende des Halses 51 vorgesehenen Anschlüsse 54a, b zu erkennen sind, die jeweils durch die durch den Nasenansatz 50 verlaufenden Kanäle verbunden sind. Die horizontale Querschnittsfläche dieser Kanäle nimmt jeweils in Richtung des unteren Endes des Halses 51 ab, so daß die horizontale Querschnittsfläche der Anschlüsse 54a, b kleiner ist als diejenige der Anschlüsse 55a, b.

Die horizontale Querschnittsfläche der Anschlüsse 55a, b entspricht dabei vorzugsweise jeweils etwa derjenigen eines Nasenlochs des zu untersuchenden Patienten. Auf diese Weise wird durch den Nasenansatz 50 dem Patienten im wesentlichen kein Strömungswiderstand entgegengesetzt, so daß dem Patienten eine praktisch vollkommen freie Atmung ermöglicht wird, die eine unverfälschte Messung des Nasenwiderstands gewährleistet. Da der Kontaktabschnitt 53 abnehmbar am Auflageabschnitt 52 gehalten ist, können entsprechend der Größe der Nasenlöcher des jeweils gerade zu untersuchenden Patienten verschiedene Kontaktabschnitte 53 mit Anschlüssen 55a, b unterschiedlich großer Querschnittsfläche verwendet werden, um auf diese Weise den Nasenansatz 50 an den jeweiligen Patienten anzupassen und somit in jedem Fall eine einwandfreie Messung sicherzustellen.

In Figur 12 ist außerdem an der dem Patienten zugewandten Seite des Nasenansatzes 50 eine Ausnehmung 57 mit einem im wesentlichen halbkreisförmigen Querschnitt zu erkennen, die im Kontaktabschnitt 53 und vorzugsweise auch im Auflageabschnitt 52 ausgebildet ist. Die Ausnehmung 57 dient zur Anpassung des Nasenansatzes 50 an den Oberlippenbereich des Patienten im angesetzten Zustand des Nasenansatzes 50.

In Figur 13 ist eine Ansicht der dem Patienten zugewandten Seite des Nasenansatzes 50 gezeigt, in der die am Kontaktabschnitt 53 und am Auflageabschnitt 52 vorgesehene Ausnehmung 57 gezeigt und die Kanäle 56a, b, die jeweils die am Kontaktabschnitt 53 vorgesehenen Anschlüsse 55a, b mit den am unteren Ende des Halses 51 ausgebildeten Anschlüssen 54a, b verbinden, durch gestrichelte Linien angedeutet sind. Um eine der Nasenform möglichst gut angepaßte Kontaktfläche zu schaffen, ist die Oberseite des Kontaktabschnitts 53 konkav ausgebildet.

Der Kontaktabschnitt 53 ist darüber hinaus vorzugsweise aus einem elastischen Material, insbesondere Schaumgummi hergestellt, so daß sich der Kontaktabschnitt 53 im angesetzten Zustand des Nasenansatzes 50 optimal an die jeweilige Form der Nase anpassen kann und eine strömungstechnisch von der Umgebung getrennte Koppelung der Nasenlöcher an die Anschlüsse 55a, b gewährleistet.

Die Benutzung des Nasenansatzes 50 im Rahmen der Rhinomanometrie wird im folgenden beschrieben.

Im Fall einer Allergiediagnose wird nach erfolgter Allergenprovokation der Nasenschleimhäute des Patienten vorzugsweise der Nasenansatz 50 auf die Anschlußhülsen des erfindungsgemäßen Aufsatzes aufgesteckt, der bevorzugt mit einem Meßkopf verbunden ist. Der Meßkopf wird an seinem Handgriff gehalten, so daß der Nasenansatz 50 von unten mit seinem Kontaktabschnitt 53 gegen die Nase des Patienten gedrückt wird und auf diese Weise die Nasenlöcher des Patienten mit den Anschlüssen 55a, b im Kontaktabschnitt 53 im wesentlichen zur Deckung kommen.

Durch den vom unteren Ende des Halses 51 zum oberen Ende des Auflageabschnitts 52 zunehmenden horizontalen Querschnitt des Nasenansatzes 50 treten weder vom Hals 51 des Nasenansatzes, noch vom Aufsatz oder vom Meßkopf störende Einflüsse am Gesicht des Patienten auf, da sich lediglich die dem Patienten zugewandte und mit der Ausnehmung 57 versehene Seite des Kontaktabschnitts 53 und vorzugsweise auch des Auflageabschnitts 52 in Kontakt mit dem Patienten befindet.

Die abnehmbare Anordnung des Kontaktabschnitts 53 ermöglicht auf vorteilhafte Weise die vorstehend bereits angeführte Anpassung des Nasenansatzes 50 an unterschiedlich große Nasenlöcher und gewährleistet ferner während der Untersuchungen hygienisch einwandfreie Bedingungen, da der Kontaktabschnitt 53 vorzugsweise als Einwegartikel konzipiert ist.

Durch den Nasenansatz 50 wird somit eine besonders einfache und bequem zu handhabende Vorrichtung zur Koppelung der Nasenlöcher des Patienten mit einer Volumenstrommeßeinrichtung und insbesondere dem erfindungsgemäßen Aufsatz geschaffen, von der ein Vorteil darin liegt, daß das normale Atmungsverhalten des Patienten bei angesetztem Nasenansatz weitestgehend unbeeinträchtigt bleibt und dadurch eine unverfälschte und genaue Messung des Nasenwiderstands gewährleistet wird.

## Patentansprüche

1. Aufsatz und Messkopf zur Verwendung in der Rhinomanometrie, wobei der Aufsatz (1) mit dem Messkopf verbindbar ist und zwei voneinander getrennt verlaufende Kanäle (8, 9) aufweist, deren eines Ende (9a, 9b) jeweils derart aus dem Aufsatz (1) austritt, dass es bei mit dem Messkopf verbundenem Aufsatz in den Messkopf mündet, und deren anderes Ende (8a, 8b) jeweils mit einem Nasenloch eines Patienten strömungstechnisch koppelbar ist, **dadurch gekennzeichnet, dass** selektiv jeweils einer der Kanäle (8, 9) versperrbar ist, so dass bei mit dem Messkopf verbundenem Aufsatz (1) nur eines der Nasenlöcher des Patienten über den jeweils nicht versperrten Kanal (8, 9) mit dem Messkopf strömungstechnisch gekoppelt ist.

2. Aufsatz nach Anspruch 1,
**dadurch gekennzeichnet,**
**dass** zur Versperrung der Kanäle (8, 9) ein Drehschieber (15) vorgesehen ist, der einen Betätigungsbereich (17) und einen sich im wesentlichen senkrecht zu den Kanälen (8, 9) erstreckenden Funktionsbereich (16) aufweist.

3. Aufsatz nach Anspruch 2,
**dadurch gekennzeichnet,**
**dass** zwei Sperrelemente (23) und zwei Öffnungen (22) am Funktionsbereich (16) des Drehschiebers (15) sowie die Kanäle (8, 9) im Aufsatz (1) so angeordnet sind, dass in einer Stellung des Drehschiebers (15) das eine Sperrelement (23) den einen Kanal (8, 9) versperrt und die eine Öffnung (22) den anderen Kanal (8, 9) freigibt, und in einer anderen Stellung das andere Sperrelement (23) bzw. die andere Öffnung (22) den jeweils anderen Kanal (8, 9) versperrt bzw. freigibt.

4. Aufsatz nach Anspruch 3,
**dadurch gekennzeichnet,**
**dass** in einer weiteren Stellung des Drehschiebers (15) beide Kanäle (8, 9) durch die Öffnungen (22) freigegeben sind.

5. Aufsatz nach Anspruch 3 oder 4,
**dadurch gekennzeichnet,**
**dass** die Öffnungen (22) und die Sperrelemente (23) im Wesentlichen kreisförmig ausgebildet sind, wobei die Durchmesser der Öffnungen (22) und der Sperrelemente (23) zumindest dem Durchmesser der Kanäle (8, 9) entsprechen.

6. Aufsatz nach einem der Ansprüche 3 bis 5,
**dadurch gekennzeichnet,**
**dass** die Sperrelemente (23) und Öffnungen (22) sowohl auf der dem Messkopf als auch der dem Patienten zugewandten Seite des Drehschiebers (15) mit umlaufenden Dichtungen (25) versehen sind.

7. Aufsatz nach Anspruch 6,
**dadurch gekennzeichnet,**
**dass** die Dichtungen (25) in im Randbereich der Sperrelemente (23) und Öffnungen (22) ausgebildeten Nuten angeordnet sind.

8. Aufsatz nach einem der Ansprüche 2 bis 7,
**dadurch gekennzeichnet,**
**dass** der Aufsatz (1) einen zylindrischen Abschnitt aufweist, in dem zur Aufnahme des Drehschiebers (15) eine senkrecht zur Zylinderachse (7) verlaufende Ausnehmung (11) vorgesehen ist, die in einer Ebene senkrecht zur Zylinderachse (7) einen Querschnitt in Form eines Kreissektors mit einem Radius entsprechend dem Zylinderradius aufweist, und der Funktionsbereich (16) des Drehschiebers (15) ebenfalls die Form eines Kreissektors mit einem Radius entsprechend dem Zylinderradius besitzt, wobei der kreissektorförmige Funktionsbereich (16) des Drehschiebers (15) einen kleineren Winkelbereich als die kreissektorförmige Ausnehmung (11) abdeckt.

9. Aufsatz nach Anspruch 8,
**dadurch gekennzeichnet,**
**dass** die Ausnehmung (11) einen zusätzlichen, im wesentlichen halbkreisförmigen Führungsausschnitt (14), dessen Mittelpunkt mit dem Mittelpunkt des die Ausnehmung (11) bildenden Kreissektors zusammenfällt, und der Funktionsbereich (16) des Drehschiebers (15) einen dem halbkreisförmigen Führungsausschnitt (14) entsprechenden Ansatz (18) aufweist, so dass der Drehschieber (15) in der Ausnehmung (11) mit seinem im Führungsausschnitt (14) gelagerten Ansatz (18) drehbar ist.

10. Aufsatz nach Anspruch 8 oder 9,
**dadurch gekennzeichnet,**
**dass** die Ausnehmung (11) einen Winkelbereich von etwa 160° und der Funktionsbereich (16) des Drehschiebers (15) einen Winkelbereich von etwa 130° abdeckt, so dass für die Drehung des Drehschiebers (15) in der Ausnehmung (11) ein Winkelbereich von etwa 30° zur Verfügung steht.

11. Aufsatz nach einem der Ansprüche 8 bis 10,
**dadurch gekennzeichnet,**
**dass** der Betätigungsbereich (17) des Drehschiebers (15) aus einem kreisbogenförmig über einen Winkelbereich von mehr als 180° gekrümmten Bandelement besteht, das sich senkrecht zum Funktionsbereich (16) des Drehschiebers (15) erstreckt und an dessen kreisbogenförmiger Seite angebracht ist, wobei sich das insbesondere flexibel ausgebildete, kreisbogenförmig mit einem dem Radius des zylindrischen Abschnitts des Aufsatzes (1) entsprechenden Radius gekrümmte Bandelement manschettenartig über einen Umfangsabschnitt des zylindrischen Abschnitts des Aufsatzes (1) erstreckt und an diesem klammerartig gehalten ist, und dass insbesondere der Betätigungsbereich (17) mit einer zur Verbesserung der Griffigkeit ausgebildeten äußeren Oberfläche versehen ist.

12. Aufsatz nach einem der vorhergehenden Ansprüche,
**dadurch gekennzeichnet,**
**dass** der Aufsatz (1) aus einem im wesentlichen zylindrischen Verbindungsteil (3) mit einem zur Verschraubung mit dem Messkopf dienenden Gewindeabschnitt (5), einem im wesentlichen zylindrischen Mittelteil (2) sowie einem sich patientenseitig an den Mittelteil (2) anschließenden und insbesondere einen Abschnitt eines Torus darstellenden Anschlussteil (4) aufgebaut ist.

13. Aufsatz nach einem der vorhergehenden Ansprüche,
**dadurch gekennzeichnet,**
**dass** patientenseitig die Kanäle (8, 9) im wesentlichen senkrecht zu ihrem Verlauf im Aufsatz (1) aus diesem austreten und am patientenseitigen Ende jeweils mit zu deren strömungstechnischer Koppelung mit den Nasenlöchern des Patienten dienenden Anschlusshülsen (12) versehen sind, die sich insbesondere im wesentlichen senkrecht zu dem Verlauf der Kanäle im Aufsatz (1) erstrecken, wobei sie im wesentlichen in einer Ebene und unter einem kleinen Winkel aufeinander zu verlaufen.

14. Aufsatz nach einem der vorhergehenden Ansprüche,
**dadurch gekennzeichnet,**
**dass** die Kanäle (8, 9) jeweils aus einem patientenseitigen Kanalabschnitt (8) und einem messkopfseitigen Kanalabschnitt (9) bestehen, die zueinander versetzt in den insbesondere im Mittelteil (2) des Aufsatzes (1) vorgesehenen, zur Versperrung der Kanäle (8, 9) dienenden Bereich münden, wobei bevorzugt jeweils der messkopfseitige Kanalabschnitt (9) versperrbar ist.

15. Aufsatz nach Anspruch 14,
**dadurch gekennzeichnet,**
**dass** ein zusätzlicher Kanal (10) vorgesehen ist, der lediglich aus einem messkopfseitigen Kanalabschnitt besteht und messkopfseitig insbesondere am Gewindeabschnitt (5) im Bereich eines Drucksensors aus dem Aufsatz (1) austritt, wobei selektiv das eine Nasenloch mit dem zusätzlichen Kanal (10) und gleichzeitig das andere Nasenloch des Patienten über einen anderen Kanal (8, 9) mit dem Messkopf strömungstechnisch koppelbar ist.

16. Aufsatz nach Anspruch 15,
**dadurch gekennzeichnet,**
**dass** der patientenseitige Abschnitt eines mittels des Drehschiebers (15) messkopfseitig jeweils versperrten Kanals (8, 9) mit dem zusätzlichen Kanal (10) strömungstechnisch gekoppelt ist.

17. Aufsatz nach Anspruch 16,
**dadurch gekennzeichnet,**
**dass** die beiden Öffnungen (22a, 22b) im Funktionsbereich (16) des Drehschiebers (15) im wesentlichen die Form eines gleichseitigen Dreiecks mit im wesentlichen kreisförmig abgerundeten Ecken (24) aufweisen, deren Krümmungsradius dem Radius der Kanäle (8a, 8b, 9a, 9b, 10) entspricht, wobei die beiden Öffnungen (22a, 22b) und die beiden Sperrelemente (23a, 23b), die versetzt angeordneten Kanalabschnitte (8a, 8b, 9a, 9b) und der zusätzliche Kanal (10) relativ zueinander so angeordnet sind, dass in der einen Drehschieberstellung der patientenseitige und der messkopfseitige Abschnitt des ersten Kanals (8a, 9a) zur strömungstechnischen Koppelung in die erste Öffnung (22a) im Bereich zweier verschiedener abgerundeter Ecken (24) münden, der patientenseitige Abschnitt des zweiten Kanals (8b) und der zusätzliche Kanal (10) zur strömungstechnischen Koppelung in die zweite Öffnung (22b) im Bereich zweier verschiedener abgerundeter Ecken (24) münden und der messkopfseitige Abschnitt des zweiten Kanals (9b) von dem zweiten Sperrelement (23b) versperrt ist, und in der anderen Drehschieberstellung der patientenseitige und der messkopfseitige Abschnitt des zweiten Kanals (8b, 9b) zur strömungstechnischen Koppelung in die zweite Öffnung (22b) im Bereich zweier verschiedener abgerundeter Ecken (24) münden, der patientenseitige Abschnitt des ersten Kanals (8a) und der zusätzliche Kanal (10) zur strömungstechnischen Koppelung in die erste Öffnung (22a) im Bereich zweier verschiedener abgerundeter Ecken (24) münden und der messkopfseitige Abschnitt des ersten Kanals (9a) von dem ersten Sperrelement (23a) versperrt ist.

18. Aufsatz nach einem der vorhergehenden Ansprüche,
**dadurch gekennzeichnet,**
**dass** der Aufsatz (1) mit einer Nasenmaske (42) koppelbar ist, die einen Maskenabschnitt (43), der mit zur Koppelung der Nasenmaske (42) an den Aufsatz (1) dienenden Anschlüssen (45a, b) versehen und an seiner der Nase eines Patienten zugewandten Seite offen ist, und einen Dichtungsabschnitt (44) umfasst, der mit dem Randbereich der offenen Seite des Maskenabschnitts (43) verbunden ist und eine Öffnung (47) aufweist, und der im aufgesetzten Zustand der Nasenmaske (42) zwischen dem Gesicht des Patienten und dem Maskenabschnitt (43) liegt, so dass die Nasenlöcher des Patienten gegen die Umgebung, nicht jedoch gegen die Anschlüsse (45a, b) abgedichtet sind, und dass im Inneren der Nasenmaske (42) eine sich im wesentlichen vertikal erstreckende und insbesondere einstückig mit dem Maskenabschnitt (43) ausgebildete Trennwand (46) vorgesehen ist, die so ausgebildet und angeordnet ist, dass die Nasenhaube (42) in jeweils eine Kammer (48a, b) für die linke und rechte Nasenhälfte unterteilt ist, in die jeweils einer der Anschlüsse (45a, b) mündet.

19. Aufsatz nach Anspruch 18,
**dadurch gekennzeichnet,**
**dass** der Dichtungsabschnitt (44) der Nasenmaske (42) auswechselbar am Maskenabschnitt (43) gehalten ist.

20. Aufsatz nach Anspruch 18 oder 19,
**dadurch gekennzeichnet,**
**dass** der Maskenabschnitt (43) der Nasenmaske (42) aus einem festen Material hoher Steifigkeit und der Dichtungsabschnitt (44) aus einem weichen Material, insbesondere Silikon hergestellt ist.

21. Aufsatz nach einem der vorhergehenden Ansprüche,
**dadurch gekennzeichnet,**
**dass** der Aufsatz (1) mit einem Nasenansatz (50) koppelbar ist, der einen Hals (51), einen auf dem Hals (51) angeordneten und vorzugsweise einstückig mit diesem ausgebildeten Auflageabschnitt (52) und einen insbesondere abnehmbar auf dem Auflageabschnitt (52) vorgesehenen Kontaktabschnitt (53) umfasst, wobei jeweils im Hals (51) bzw. im Kontaktabschnitt (53) Anschlüsse (54a, b) (55a, b) zur Koppelung des Nasenansatzes (50) an den Aufsatz (1) bzw. mit den Nasenlöchern eines Patienten vorgesehen sind, die durch im wesentlichen in vertikaler Richtung durch den Nasenansatz (50) verlaufende Kanäle (56a, b) verbunden sind.

22. Aufsatz nach Anspruch 21,
**dadurch gekennzeichnet,**
**dass** der Kontaktabschnitt (53) des Nasenansatzes (50) aus einem elastischen Material, insbesondere Schaumgummi hergestellt ist.

23. Aufsatz nach Anspruch 21 oder 22,
**dadurch gekennzeichnet,**
**dass** der Kontaktabschnitt (53) des Nasenansatzes (50) ein Einwegartikel ist.

24. Aufsatz nach einem der Ansprüche 21 bis 23,
**dadurch gekennzeichnet,**
**dass** der Hals (51) und der Auflageabschnitt (52) des Nasenansatzes (50) einstückig miteinander ausgebildet sind und die horizontale Querschnittsfläche der durch den Hals (51) und den Auflageabschnitt (52) gebildeten Anordnung vom unteren Ende des Halses (51) zum oberen Ende des Auflageabschnitts (52) so zunimmt, dass ein Abstand zwischen dem Patienten und dem Hals (51) gegeben ist und gleichzeitig die dem Patienten zugewandte Seite des Kontaktabschnitts (53) und vorzugsweise auch des Auflageabschnitts (52) am Oberlippenbereich des Patienten angesetzt werden kann.

## Claims

1. Fixture and measuring head for use in rhinomanometry, wherein the fixture (1) is connectable to the measuring head and has two passages (8, 9) extending separately from one another the one end of which (9a, 9b) respectively leads out of the fixture (1) in such a way that it opens into the measuring head when the fixture is connected to the measuring head and the other end (8a, 8b) of which is respectively flow-technically coupleable to a nostril of a patient,
**characterized in that**
one of the passages (8, 9) can be selectively blocked so that with the fixture (1) connected to the measuring head only one of the nostrils of the patient is flow-technically coupled to the measuring head via the respectively non-blocked passage (8, 9).

2. Fixture in accordance with claim 1,
**characterized in that**
for the blocking of the passages (8, 9) a rotary gate (15) is provided which has an actuating region (17) and a functional region (16) extending substantially perpendicular to the passages (8, 9).

3. Fixture in accordance with claim 2,
**characterized in that**
two blocking elements (23) and two openings (22) are so arranged at the functional region (16) of the rotary gate (15) and also the passages (8, 9) are so arranged in the fixture (1) that in one position of the rotary gate (15) the one blocking element (23) blocks the one passage (8, 9) and the one opening (22) frees the other passage (8, 9) and in the other position the other blocking element (23) and the other opening (22) respectively block and free the other respective passage (8, 9).

4. Fixture in accordance with claim 3,
**characterized in that**
in a further position of the rotary gate (15) both passages (8, 9) are freed by the openings (22).

5. Fixture in accordance with claim 3 or claim 4,
**characterized in that**
the openings (22) and the blocking elements (23) are of substantially circular shape, with the diameter of the openings (22) and of the blocking elements (23) corresponding at least to the diameter of the passages (8, 9).

6. Fixture in accordance with one of the claims 3 to 5,
**characterized in that**
the blocking elements (23) and the openings (22) are provided with peripherally extending seals (25) both at both the side of the rotary gate facing the measuring head and also at the side facing the patient.

7. Fixture in accordance with claim 6,
**characterized in that**
the seals (25) are arranged in grooves formed in the marginal region of the blocking elements (23) and openings (22).

8. Fixture in accordance with one of the claims 2 to 7,
**characterized in that**
the fixture (1) has a cylindrical section in which a cut-out (11) extending perpendicular to the cylinder axis (7) is provided to receive the rotary gate (15), the cut-out (11) having a cross-section in the form of a circular sector with a radius corresponding to the cylinder radius in a plane perpendicular to the cylinder axis (7) and **in that** the functional region (16) of the rotary gate (15) likewise has the form of a circular sector with a radius corresponding to the cylinder radius, with the circular sector-like functional region (16) of the rotary gate (15) extending over a smaller angular range than the circular sector-like cut-out (11).

9. Fixture in accordance with claim 8,
**characterized in that**
the cut-out (11) has an additional semi-circular guide cut-out (14) the centre point of which coincides with the centre point of the circular sector forming the cut-out (11) and the functional region (16) of the rotary gate (15) has a formation corresponding to the semi-circular guide cut-out (14) so that the rotary gate (15) is rotatable in the cut-out (11) with its formation (18) journalled in the guide cut-out (14).

10. Fixture in accordance with claim 8 or claim 9,
**characterized in that**
the cut-out (11) extends over an angular region of approximately 160° and the functional region (16) of the rotary gate (15) extends over an angular range of approximately 130° so that an angular range of approximately 30° is available for the rotation of the rotary gate (15) in the cut-out (11).

11. Fixture in accordance with one of the claims 8 to 10,
**characterized in that**
the actuating region (17) of the rotary gate (15) consists of a circular arc-shape band element curved over an angular range of more than 180° which extends perpendicular to the functional region (16) of the rotary gate (15) and is attached to its circular arc-shaped side, with the band element, which is in particular flexibly formed and curved in a circular arc-shape with a radius corresponding to the radius of the cylindrical section of the fixture (1), extending in cuff-like manner over a peripheral section of the cylindrical section of the fixture (1) and being held in clip-like manner on the latter, and **in that** the actuating region in particular is provided with an outer surface formed to improve the grip.

12. Fixture in accordance with one of the preceding claims,
**characterized in that**
the fixture (1) is build-up of a substantially cylindrical connection part (3) with a thread section (5) serving for the screw connection to the measuring head, of a substantially cylindrical centre part (2) and also a connection part (4) which adjoins the centre part (2) at the patient side and in particular represents a section of a toroid.

13. Fixture in accordance with one of the preceding claims,
**characterized in that**
the passages (8, 9) at the patient side emerge out of the fixture (1) substantially perpendicular to their course in the fixture (1) and are respectively provided at the patient side end with connection sleeves (12) serving for the coupling to the nostrils of the patient, the connection sleeves (12) in particular extending substantially perpendicular to the course of the passages in the fixture (1) essentially in a plane and converging at a small angle.

14. Fixture in accordance with one of the preceding claims,
**characterized in that**
the passages (8, 9) each consist of a patient side passage section (8) and a measuring head side passage section (9) which open offset to one another into the region which serves for the blocking of the passages (8, 9) and which is in particular provided in the middle part (2) of the fixture (1), with the measuring head side passage section (9) preferably being blockable in each case.

15. Fixture in accordance with claim 14,
**characterized in that**
an additional passage (10) is provided which only consists of a measuring head side passage section and which emerges at the measuring head side out of the fixture (1) in particular at the threaded section (5) in the region of a pressure sensor, with the one nostril being selectively couplable with the additional passage (10) and the other nostril of the patient simultaneously being flow-technically couplable with the measuring head via another passage (8, 9).

16. Fixture in accordance with claim 15,
**characterized in that**
the patient side section of a passage (8, 9) which is respectively blocked at the measuring head side by means of the rotary gate is flow-technically coupled to the additional passage (10).

17. Fixture in accordance with claim 16,
**characterized in that**
the two openings (22a, 22b) in the functional region (16) of the rotary gate (15) have substantially the shape of an equilateral triangle with substantially circularly rounded corners (24) the radius of curvature of which corresponds to the radius of the passages (8a, 8b, 9a, 9b, 10), with the two openings (22a, 22b) and the two blocking elements (23a, 23b), the offset passage sections (8a, 8b, 9a, 9b) and the additional passage (10) being so arranged relative to one another that in the one position of the rotary gate the patient side section and the measuring head side section of the first passage (8a, 9a) open for the flow-technical coupling into the first opening (22a) in the region of two different rounded corners (24), the patient side section of the second passage (8b) and the additional passage (10) open for the flow-technical coupling into the second opening (22b) in the region of two different rounded corners (24) and the measuring head side section of the second passage (9b) is blocked by the second blocking element (23b) and, in the other position of the rotary gate, the patient side section and the measuring head side section of the second passage (8b, 9b) open for the flow-technical coupling into the second opening (22b) in the region between two different rounded corners (24), the patient side section of the first passage (8a) and the additional passage (10) open for the flow-technical coupling into the first opening (22a) in the region of two different rounded corners (24) and the measuring head side section of the first passage (9a) is blocked by the first blocking element (23a).

18. Fixture in accordance with one of the preceding claims,
**characterized in that**
the fixture (1) can be coupled to a nose mask (42) which is provided with a mask section (43), the mask section (43) being provided with connections (45a, b) serving for the coupling of the nose mask (42) to the fixture (1), being open at its side facing the nose of the patient and including a sealing section (44) which is connected to the marginal zone of the open side of the mask section (43), has an opening (47) and, in the mounted state of the nose mask (42), lies between the face of the patient and the mask section (43) so that the nostrils of the patient are sealed relative to the environment but not however relative to the connections (45a, b), and **in that** a substantially vertically extending partition wall (46), which is in particular formed in one piece with the mask section (43) is provided in the interior of the nose mask (42) and is so designed and arranged that the nose hood (42) is respectively subdivided into a chamber (48a, b) for the left and right halves of the nose into which a respective one of the connections (45a, b) opens.

19. Fixture in accordance with claim 18,
**characterized in that**
the sealing section (44) of the nose mask (42) is interchangeably held at the mask section (43).

20. Fixture in accordance with claim 18 or 19,
**characterized in that**
the mask section (43) of the nose mask (42) is manufactured from a solid material of high stiffness and the sealing section (44) is manufactured of a soft material, in particular of silicone.

21. Fixture in accordance with one of the preceding claims,
**characterized in that**
the fixture (1) can be coupled to a nose fitting (50) which includes a neck (51), a support section (52) arranged on the neck (51) and preferably formed in one piece with it and a contact section (53) which is in particular removably provided on the support section (52), with connections (54a, b) (55a, b) being respectively provided in the neck (51) and in the contact section (53) for the coupling of the nose fitting (50) to the fixture (1) and to the nostrils of a patient and which are connected by passages (56a, b) extending substantially in the vertical direction through the nose fitting (50).

22. Fixture in accordance with claim 21,
**characterized in that**
the contact section (53) of the nose fitting (50) is manufactured from an elastic material, in particular foam rubber.

23. Fixture in accordance with claim 21 or 22,
**characterized in that**
the contact section (53) of the nose fitting (50) is a disposable article.

24. Fixture in accordance with one of the claims 21 to 23,
**characterized in that**
the neck (51) and the support section (52) of the nose fitting (50) are formed in one piece with one another and the horizontal cross-sectional surface of the arrangement formed by the neck (51) and the support section (52) so increases from the lower end of the neck (51) to the upper end of the support section (52) that a spacing is present between the patient and the neck (51) and the side of the contact section (53) confronting the patient and preferably also the support section (52) can be placed on the upper lip region of the patient.

## Revendications

1. Accessoire de connexion et tête de mesure pour l'utilisation en rhinomanométrie, l'accessoire de connexion (1) pouvant être relié à la tête de mesure et comprenant deux canaux (8, 9) s'étendant séparément l'un de l'autre dont une extrémité (9a, 9b) sort hors de l'accessoire de connexion (1) de telle sorte qu'elle débouche dans la tête de mesure, l'accessoire de connexion étant relié à la tête de mesure, et dont l'autre extrémité (8a, 8b) peut être couplée en écoulement à une narine d'un patient, caractérisé(e) en ce que l'un des canaux respectifs (8, 9) peut être obturé sélectivement, de sorte que, l'accessoire de connexion (1) étant relié à la tête de mesure, une seule des narines du patient est couplée en écoulement avec la tête de mesure via le canal respectif non obturé (8, 9).

2. Accessoire de connexion selon la revendication 1, **caractérisé en ce que** pour obturer les canaux (8, 9), il est prévu un tiroir rotatif (15) qui comprend une zone d'actionnement (17) et une zone fonctionnelle (16) qui s'étend sensiblement perpendiculairement aux canaux (8, 9).

3. Accessoire de connexion selon la revendication 2, **caractérisé en ce que** deux éléments d'obturation (23) et deux ouvertures (22) sont prévus au niveau de la zone fonctionnelle (16) du tiroir rotatif (15), et les canaux (8, 9) sont prévus dans l'accessoire de connexion (1), de telle sorte que dans une position du tiroir rotatif (15) l'un des éléments d'obturation (23) obture l'un des canaux (8, 9) et l'une des ouvertures (22) libère l'autre canal (8, 9), et dans une autre position l'autre élément d'obturation (23) et l'autre ouverture (22) obture et libère respectivement l'autre canal respectif (8, 9).

4. Accessoire de connexion selon la revendication 3, **caractérisé en ce que** dans une autre position du tiroir rotatif (15), les deux canaux (8, 9) sont libérés par les ouvertures (22).

5. Accessoire de connexion selon l'une ou l'autre des revendications 3 et 4, **caractérisé en ce que** les ouvertures (22) et les éléments d'obturation (23) sont réalisés sensiblement en forme circulaire, les diamètres des ouvertures (22) et des éléments d'obturation (23) correspondant au moins au diamètre des canaux (8, 9).

6. Accessoire de connexion selon l'une des revendications 3 à 5, **caractérisé en ce que** les éléments d'obturation (23) et les ouvertures (22) sont pourvus de joints d'étanchéité périphériques (25) aussi bien sur le côté du tiroir rotatif (15), qui est tourné vers la tête de mesure, que sur le côté tourné vers le patient.

7. Accessoire de connexion selon la revendication 6, **caractérisé en ce que** les joints (25) sont agencés dans des gorges ménagées dans la zone marginale des éléments d'obturation (23) et des ouvertures (22).

8. Accessoire de connexion selon l'une des revendications 2 à 7, **caractérisé en ce que** l'accessoire de connexion (1) comprend un tronçon cylindrique dans lequel est prévu un évidement (11) s'étendant perpendiculairement à l'axe de cylindre (7) pour recevoir le tiroir rotatif (15), évidement qui présente dans un plan perpendiculaire à l'axe de cylindre (7) une section en forme de secteur de cercle d'un rayon correspondant au rayon du cylindre, et **en ce que** la zone fonctionnelle (16) du tiroir rotatif (15) possède également la forme d'un secteur de cercle avec un rayon correspondant au rayon du cylindre, la zone fonctionnelle (16) en forme de secteur de cercle du tiroir rotatif (15) couvrant une plage angulaire plus petite que l'évidement (11) en forme de secteur de cercle.

9. Accessoire de connexion selon la revendication 8, **caractérisé en ce que** l'évidement (11) comprend une échancrure de guidage supplémentaire (14) sensiblement en forme de demi-cercle dont le centre coïncide avec le centre du secteur de cercle formant l'évidement (11), et **en ce que** la zone fonctionnelle (16) du tiroir rotatif (15) comprend un talon (18) correspondant à l'échancrure de guidage (14) en forme de demi-cercle, de sorte que le tiroir rotatif (15) est mobile en rotation dans l'évidement (11) par son talon (18) monté dans l'échancrure de guidage (14).

10. Accessoire de connexion selon l'une ou l'autre des revendications 8 et 9, **caractérisé en ce que** l'évidement (1) couvre une plage angulaire d'environ 160° et la zone fonctionnelle (16) du tiroir rotatif (15) couvre une plage angulaire d'environ 130°, de sorte qu'une plage angulaire d'environ 30° est disponible pour la rotation du tiroir rotatif (15) dans l'évidement (11).

11. Accessoire de connexion selon l'une des revendications 8 à 10, **caractérisé en ce que** la zone d'actionnement (17) du tiroir rotatif (15) est constituée par un élément de bande recourbé en forme d'arc de cercle sur une zone angulaire de plus de 180°, qui s'étend perpendiculairement à la zone fonctionnelle (16) du tiroir rotatif (15) et qui est agencé à son côté en forme d'arc de cercle, et l'élément de bande réalisé en particulier flexible et recourbé en forme d'arc de cercle dont le rayon correspond au rayon du tronçon cylindrique de l'accessoire de connexion (1) s'étend à la manière d'une manchette sur une partie périphérique du tronçon cylindrique de l'accessoire de connexion (1) et est maintenu sur celui-ci à la manière d'une pince, et **en ce qu'**en particulier la zone d'actionnement (17) est réalisée avec une surface extérieure réalisée pour améliorer l'adhérence.

12. Accessoire de connexion selon l'une des revendications précédentes, **caractérisé en ce que** l'accessoire de connexion (1) est constitué par une partie de liaison sensiblement cylindrique (3) comportant un tronçon à pas de vis (5) servant au vissage avec la tête de mesure, par une partie médiane sensiblement cylindrique (2) ainsi que par une partie de raccordement (4) qui se raccorde du côté patient à la partie médiane (2) et qui représente en particulier un tronçon de tore.

13. Accessoire de connexion selon l'une des revendications précédentes, **caractérisé en ce que** du côté patient les canaux (8, 9) sortent hors de l'accessoire de connexion (1) sensiblement perpendiculairement à leur tracé dans celui-ci et sont pourvus, à l'extrémité respective côté patient, de douilles de raccordement (12) servant à leur couplage en écoulement avec les narines du patient, douilles qui s'étendent en particulier sensiblement perpendiculairement au tracé des canaux dans l'accessoire de connexion (1) en convergeant sensiblement dans un plan et sous un petit angle.

14. Accessoire de connexion selon l'une des revendications précédentes, **caractérisé en ce que** les canaux (8, 9) sont constitués chacun par un tronçon de canal (8) côté patient et par un tronçon de canal (9) côté tête de mesure qui débouchent en décalage mutuel dans la zone agencée en particulier dans la partie médiane (2) de l'accessoire de connexion (1) et servant à l'obturation des canaux (8, 9), de préférence le tronçon de canal respectif (9) côté patient étant obturable.

15. Accessoire de connexion selon la revendication 14, **caractérisé en ce qu'**il est prévu un canal supplémentaire (10) qui est constitué uniquement par un tronçon de canal côté tête de mesure et qui sort hors de l'accessoire de connexion (1) du coté tête de mesure en particulier au niveau du tronçon à pas de vis (5) dans la zone d'un capteur de pression, dans lequel sélectivement l'une des narines peut être couplée en écoulement avec le canal supplémentaire (10) et simultanément l'autre narine du patient peut être couplée en écoulement avec la tête de mesure via un autre canal (8, 9).

16. Accessoire de connexion selon la revendication 15, **caractérisé en ce que** le tronçon côté patient d'un canal respectif (8, 9) obturé du côté tête de mesure au moyen du tiroir rotatif (15) est couplé en écoulement avec le canal supplémentaire (10).

17. Accessoire de connexion selon la revendication 16, **caractérisé en ce que** les deux ouvertures (22a, 22b) dans la zone fonctionnelle (16) du tiroir rotatif (15) présentent sensiblement la forme d'un triangle équilatéral à coins (24) arrondis sensiblement en forme circulaire, dont le rayon de courbure correspond au rayon des canaux (8a, 8b, 9a, 9b, 10), les deux ouvertures (22a, 22b) et les deux éléments d'obturation (23a, 23b), les tronçons de canal agencés en décalage (8a, 8b, 9a, 9b) et le canal supplémentaire (10) étant agencés les uns par rapport aux autres de telle sorte que dans l'une des positions du tiroir rotatif le tronçon côté patient et le tronçon côté tête de mesure du premier canal (8a, 9a) débouchent, pour le couplage en écoulement, dans la première ouverture (22a) dans la zone de deux coins arrondis différents (24), le tronçon côté patient du deuxième canal (8b) et le canal supplémentaire (10) débouchent, pour le couplage en écoulement, dans la deuxième ouverture (22b) dans la zone de deux coins arrondis différents (24), et le tronçon côté tête de mesure du deuxième canal (9b) est obturé par le deuxième élément d'obturation (23b), et dans l'autre position du tiroir rotatif le tronçon côté patient et le tronçon côté tête de mesure du deuxième canal (8b, 9b) débouchent, pour le couplage en écoulement, dans la deuxième ouverture (22b) dans la zone de deux coins arrondis différents (24), le tronçon côté patient du premier canal (8a) et le canal supplémentaire (10) débouchent, pour le couplage en écoulement, dans la première ouverture (22a) dans la zone de deux coins arrondis différents (24), et le tronçon côté tête de mesure du premier canal (9a) est obturé par le premier élément d'obturation (23a).

18. Accessoire de connexion selon l'une des revendications précédentes, **caractérisé en ce que** l'accessoire de connexion (1) est susceptible d'être couplé avec un masque nasal (42) qui comprend un tronçon de masque (43) qui est pourvu de raccords (45a, b) servant au couplage du masque nasal (42) avec l'accessoire de connexion (1) et qui est ouvert à son côté tourné vers le nez d'un patient, et un tronçon d'étanchement (44) est qui relié à la zone marginale du côté ouvert du tronçon de masque (43) et qui présente une ouverture (47), et qui se trouve, dans l'état mis en place du masque nasal (42), entre le visage du patient et le tronçon de masque (43), de telle sorte que les narines du patient sont étanchées par rapport à l'environnement, mais non pas par rapport aux raccords (45a, b), et **en ce qu'**il est prévu dans l'intérieur du masque nasal (42) une paroi de séparation (46) qui s'étend sensiblement verticalement et qui est réalisée en particulier d'un seul tenant avec le tronçon de masque (43), paroi qui est réalisée et agencée de telle sorte que la coiffe nasale (42) est subdivisée en une chambre (48a, b) pour la moitié de nez de gauche et pour la moitié de nez de droite, dans laquelle débouche l'un des raccords respectifs (45a, b).

19. Accessoire de connexion selon la revendication 18, **caractérisé en ce que** le tronçon d'étanchéité (44) du masque nasal (42) est maintenu de façon interchangeable sur le tronçon de masque (43).

20. Accessoire de connexion selon l'une ou l'autre des revendications 18 et 19, **caractérisé en ce que** le tronçon (43) du masque nasal (42) est réalisé en matériau solide de haute rigidité, et le tronçon d'étanchéité (44) est réalisé en matériau souple, en particulier de silicone.

21. Accessoire de connexion selon l'une des revendications précédentes, **caractérisé en ce que** l'accessoire de connexion (1) est susceptible d'être couplé à un talon nasal (50) qui comprend un col (51), un tronçon d'appui (52) agencé sur le col (51) et réalisé de préférence d'un seul tenant avec celui-ci, et un tronçon de contact (53) prévu en particulier de façon amovible sur le tronçon d'appui (52), des raccords (54a, b) (55a, b) pour le couplage du talon nasal (50) sur l'accessoire de connexion (1) ou avec les narines d'un patient étant prévus dans le col (51) ou dans le tronçon de contact (53), raccords qui sont reliés par des canaux (56a, b) s'étendant sensiblement en direction verticale à travers le talon nasal (50).

22. Accessoire de connexion selon la revendication 21, **caractérisé en ce que** le tronçon de contact (53) du talon nasal (50) est réalisé en matériau élastique, en particulier en caoutchouc mousse.

23. Accessoire de connexion selon l'une ou l'autre des revendications 21 et 22, **caractérisé en ce que** le tronçon de contact (53) du talon nasal (50) est un article à usage unique.

24. Accessoire de connexion selon l'une des revendications précédentes, **caractérisé en ce que** le col (51) et le tronçon d'appui (52) du talon nasal (50) sont réalisés d'un seul tenant, et la surface de section horizontale de l'agencement formé par le col (51) et par le tronçon d'appui (52) augmente depuis l'extrémité inférieure du col (51) vers l'extrémité supérieure du tronçon d'appui (52), de telle sorte qu'il existe une distance entre le patient et le col (51) et que le côté du tronçon de contact (53) qui est détourné du patient et de préférence également celui du tronçon d'appui (52) peuvent être posés sur la zone de la lèvre supérieure du patient.
